# EUROPEAN PATENT SPECIFICATION

(11) **EP 1 673 022 B1**
(45) Date of publication and mention of the grant of the patent: **11.04.2012**
(21) Application number: 04748105.6
(22) Date of filing: 23.07.2004
(51) Int. Cl.: A61B 17/28, A61B 17/29

(54) **FORCEPS COVER SHEATH, SURGICAL FORCEPS AND SURGICAL FORCEPS SYSTEM**
ZANGEN-ABDECK-HÜLLE, CHIRURGISCHE ZANGE UND CHIRURGISCHES ZANGENSYSTEM
GAINE DE RECOUVREMENT DE PINCE, PINCE CHIRURGICALE ET SYSTEME DE PINCE CHIRURGICALE

(30) Priority: 24.07.2003 JP 2003201066
(43) Date of publication of application: 28.06.2006
(73) Proprietor: Olympus Corporation, Tokyo 151-0072 (JP)
(72) Inventor: OGAWA, Akihisa, 2-3 Kuboyama,Hachioji-shi Tokyo 192-8512 (JP); TSURUO, Hatori, 2-3 Kuboyama,Hachioji-shi Tokyo 192-8512 (JP)
(74) Representative: von Hellfeld, Axel
(86) International application number: PCT/JP2004/010898
(87) International publication number: WO 2005/009255

(56) References cited:
- US-A- 3 022 787
- US-A- 4 834 090
- US-A- 5 036 733
- US-A- 5 496 347
- US-A1- 2001 039 433
- US-B1- 6 261 275
- US-B1- 6 273 887

## Description

### Technical Field

The present invention relates to a forceps cover sheath, attached to a forceps, for example, for surgical use, a surgical forceps and a surgical forceps system.

### Background Art

Various kinds of forcipes are used, for example, in surgical operations under endoscopes. A surgical forceps generally includes an elongated insertion section (shaft) and an operating section provided at a proximal end portion of the insertion section.

A treatment portion having a link mechanism is provided at a distal end portion of the insertion section. The forceps is operated in conjunction with the link mechanism when the link mechanism is moved by operation of the operating section.

Such a surgical forceps is, during its use, inserted into a treatment target (body). Various stains such as blood and body fluids from the treatment target remain on the treatment portion (link mechanism) of the insertion section after the forceps is used. Thus, the surgical forceps is, after surgery (use), sterilized through processes such as cleaning, disinfection and sterilization for reuse in a subsequent operation.

For example, Jpn. Pat. Appln. KOKAI Publication No. 10-71155 discloses a technique which separates the surgical forceps into a plurality of assemblies to enable easier cleaning of the link mechanism. The surgical forceps is separated into a sheath assembly, a treatment portion drive assembly part of which is inserted in the sheath assembly, and an operating assembly to operate the treatment portion drive assembly. The treatment portion drive assembly is inserted in the sheath assembly, and provided with the link mechanism between an operating shaft coupled to an operating section assembly and the treatment portion. The link mechanism is covered with the sheath assembly during use. As the link mechanism is exposed when the forceps is disassembled, it is easy to clean and remove stains.

When the surgical forceps disclosed in Jpn. Pat. Appln. KOKAI Publication No. 10-71155 is cleaned, the link mechanism, in particular, needs to be elaborately cleaned after the forceps is disassembled. The cleaning requires time and labor.

For example, in the case of a link mechanism which is more complex than that of the surgical forceps disclosed in Jpn. Pat. Appln. KOKAI Publication No. 10-71155, the time and labor required to clean the link mechanism are increased with the complexity of the link mechanism. The surgical forceps can be disposable, but this leads to much increased medical expenses.

US 2001/0039433 discloses a surgical instrument jaw cover having a bridging member disposed between two shods, in order to prevent tissue from becoming stuck in a hinge of the surgical instrument on which it is disposed.

US 3 022 787 discloses the features of the preamble of claim 1 and describes a tool for rectal treatment comprising two spoon shaped blades disposed on a surgical clamp, whereby, when the clamp is opened, the spoon shaped blades open to receive fecal matter. Additionally, a tubular guard or sleeve, comprised of rubber or other soft resilient material, is disposed over a pivot portion of the surgical clamp, in order to prevent tissue from contacting the pivot portion of the surgical clamp, and to maintain the instrument in a clean condition.

### Disclosure of Invention

The invention is defined in the appended claims and has been attained to solve such problems, and has an object to provide a forceps cover sheath, a surgical forceps and a surgical forceps system capable of reducing time and labor to clean the forceps after its use.

Advantages of the invention will be set forth in the description which follows, and in part will be obvious from the description, or may be learned by practice of the invention. The objects and advantages of the invention may be realized and obtained by means of the instrumentalities and combinations particularly pointed out hereinafter.

### Brief Description of Drawings

The accompanying drawings, which are incorporated in and constitute a part of the specification, illustrate embodiments of the invention, and together with the general description given above and the detailed description of the embodiments given below, serve to explain the principles of the invention.
FIG. 1A is a schematic side view of a forceps according to a first example ;
FIG. 1B is a side view showing the vicinity of a distal end portion of the forceps in FIG. 1A according to the first example ;
FIG. 2A is a schematic diagram showing a sheath assembly when the forceps according to the first example is disassembled;
FIG. 2B is a schematic diagram showing a treatment portion drive assembly when the forceps is disassembled according to the first example ;
FIG. 2C is a schematic diagram showing an operating section assembly when the forceps is disassembled according to the first example ;
FIG. 3A is a perspective view of a forceps cover sheath attached to the forceps according to the first example;
FIG. 3B is an outline view from an arrow 3B direction in FIG. 3A;
FIG. 3C is a schematic perspective view showing a distal end portion of the forceps cover sheath;
FIG. 3D is a schematic perspective view showing the distal end portion of the forceps cover sheath;
FIG. 3E is a schematic diagram showing when the forceps is inserted into an inner space of the forceps cover sheath;
FIG. 4 is a schematic diagram showing when the forceps cover sheath is attached to the forceps according to the first example ;
FIG. 5 is a schematic perspective view of a modification of a grab of the forceps cover sheath attached to the forceps according to the first example;
FIG. 6A is a schematic perspective view of the distal end of the forceps cover sheath attached to the forceps according to an embodiment;
FIG. 6B is a schematic diagram showing openings at the distal end of the forceps cover sheath attached to the forceps according to the embodiment as observed from an arrow 6B direction in FIG. 6A;
FIG. 6C is a schematic diagram showing the forceps and the distal end of the forceps cover sheath in which the forceps cover sheath is attached to the forceps according to the embodiment;
FIG. 7A is a schematic diagram showing the sheath assembly when the forceps according to a second example is disassembled;
FIG. 7B is a schematic diagram showing the treatment portion drive assembly when the forceps is disassembled according to the second example;
FIG. 7C is a schematic diagram showing the operating section assembly when the forceps is disassembled according to the second example;
FIG. 7D is a schematic diagram showing the forceps cover sheath to cover the treatment portion drive assembly from its distal end portion when the treatment portion drive assembly is attached to the sheath assembly;
FIG. 8A is a schematic diagram showing a link mechanism at a distal end portion of an insertion section of the forceps according to the second example;
FIG. 8B is, above a central line, a schematic outline view of the forceps cover sheath including a treatment portion component attached to the distal end of the link mechanism, and below central line, a longitudinal sectional view thereof;
FIG. 8C is a schematic diagram showing the distal end portion of the treatment portion when the forceps cover sheath is attached to the forceps;
FIG. 9 is a schematic diagram showing the treatment portion component before being connected to the distal end of the link mechanism in the forceps according to the second example;
FIG. 10 is a schematic partial sectional view of the vicinity of the operating section of the forceps according to the second example;
FIG. 11A is a schematic diagram showing a modification of the forceps according to the second example, and showing an attachment aid which holds a proximal end portion of the forceps cover sheath;
FIG. 11B is a schematic diagram showing when the forceps cover sheath is stored in a state attached to the attachment aid shown in FIG. 11A;
FIG. 11C is a schematic diagram showing a modification of the attachment aid shown in FIG. 11A;
FIG. 12A is a schematic diagram of the forceps according to a third example;
FIG. 12B is a schematic diagram showing a water conveyance system coupled to a cleaning port of the forceps shown in FIG. 12A;
FIG. 13A is a schematic diagram showing a holding portion in which a physiological saline pack of the water conveyance system used with the forceps according to the third example is to be disposed;
FIG. 13B is an upper view of the holding portion shown in FIG. 13A;
FIG. 14A is a side view of the forceps in a straight state according to a fourth example;
FIG. 14B is a side view of the treatment portion and the operating section in a pivoted state;
FIG. 14C is a bottom view of the distal end portion of the insertion section; and
FIG. 15 is a schematic diagram showing a configuration of the distal end portion of the forceps according to the fourth example.

### Best Mode for Carrying Out the Invention

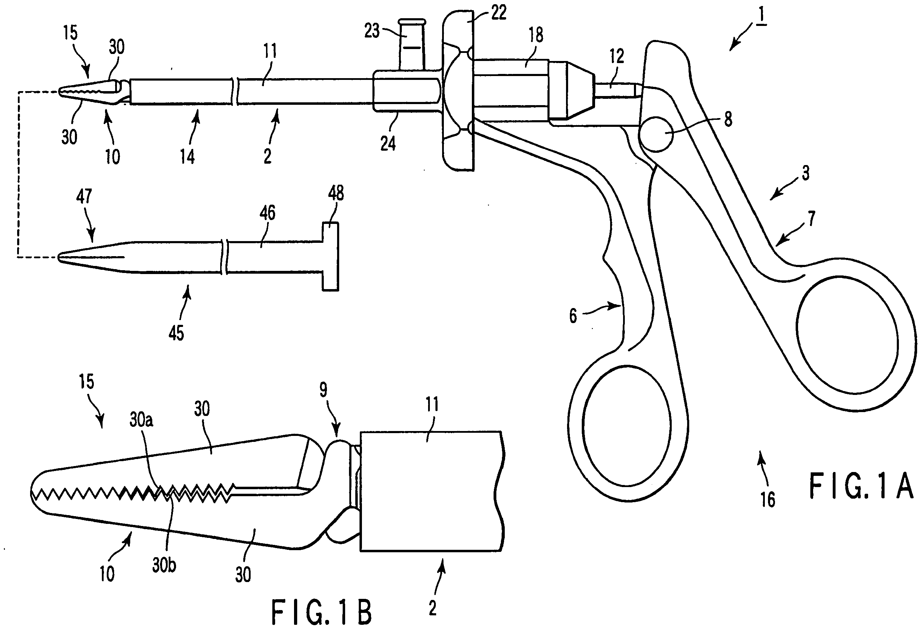

Embodiments of the present invention will hereinafter be described in reference to the drawings. A first example will be described referring to FIG. 1A to FIG. 6C.

FIG. 1A shows a schematic configuration of a surgical forceps 1. As shown in FIG. 1A, the surgical forceps 1 includes an elongated insertion section 2 to be inserted into a treatment target (body), and an operating section 3 at hand coupled to a proximal end portion of the insertion section 2.

The operating section 3 includes a fixed handle 6, and a movable handle 7 which can move relatively with the fixed handle 6. The movable handle 7 is pivotally coupled to the fixed handle 6 by a rotation shaft 8 for the movable handle 7 to pivot with respect to the fixed handle 6.

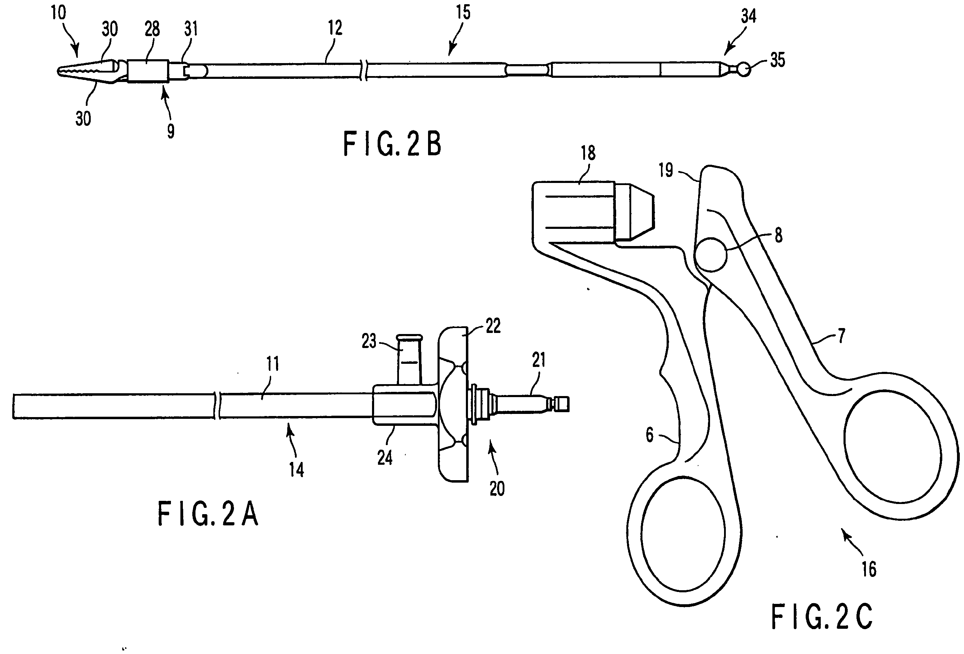

A treatment portion 10 having a link mechanism (open-close drive mechanism) 9 shown in FIG. 2B is provided at a distal end portion of the insertion section 2. The insertion section 2 includes an elongated cylindrical sheath 11 shown in FIG. 2A. An operating shaft 12 shown in FIG. 2B which is connected to the treatment portion 10 is movably provided in the sheath 11. If the movable handle 7 is pivoted with respect to the fixed handle 6 on the rotation shaft 8 of the operating section 3, the operating shaft 12 advances or retracts in an axial direction of the insertion section 2. The link mechanism 9 of the treatment portion 10 is driven along with the advancing or retracting of the operating shaft 12.

The forceps 1 includes a sheath assembly 14 shown in FIG. 2A, a treatment portion drive assembly 15 shown in FIG. 2B, and an operating section assembly 16 shown in FIG. 2C. The operating section assembly 16 shown in FIG. 2C constitutes the operating section 3 described above. A sheath coupling member 18 which detachably couples the sheath assembly 14 is provided at an upper end portion of the fixed handle 6 of the operating section assembly 16. An operating shaft coupling portion 19 which detachably couples the operating shaft 12 of the treatment portion drive assembly 15 is provided at an upper end portion of the movable handle 7.

The sheath assembly 14 shown in FIG. 2A constitutes an outer envelope of the insertion section 2. The sheath assembly 14 includes the sheath 11 and a connection portion 20 to be connected to the operating section assembly 16 at the proximal end portion of the sheath 11. The connection portion 20 includes a handle coupling portion 21, a rotating knob 22, and a cleaning port 23. The handle coupling portion 21 is detachably coupled to the sheath coupling member 18 of the fixed handle 6 of the operating section assembly 16. The rotating knob 22 rotates the sheath 11. The cleaning port 23 protrudes on a cylindrical exterior 24 attached to an outer peripheral surface of a proximal end portion side of the sheath 11.

As shown in FIG. 2B, the treatment portion drive assembly 15 includes the treatment portion 10 having the link mechanism 9, and the operating shaft 12 provided at a proximal end of the treatment portion 10. A holding member 28 which constitutes the link mechanism 9 of the treatment portion 10 is provided at a distal end of the operating shaft 12. A pair of jaws 30 is provided at a distal end of the holding member 28. In case that the operating shaft 12 moves forward and backward along an axial direction of the operating shaft 12 itself, the pair of jaws 30 is driven to relatively open and close by the link mechanism 9.

As shown in FIG. 1B, teeth 30a, 30b are respectively formed on surfaces that contact each other when the jaws 30 are closed. Thus, a gripped region (tissue) when the treatment target is gripped by the teeth 30a, 30b of the jaws 30 is prevented from slipping.

As shown in FIG. 2B, a connection portion 31 to the sheath assembly 14 is provided at a rear end of the holding member 28. The connection portion 31 is inserted into the sheath 11 of the sheath assembly 14, and is engaged or disengaged in the sheath 11. The treatment portion drive assembly 15 and the sheath assembly 14 are engaged to each other and coupled.

A connection portion 34 to be connected to the movable handle 7 of the operating section 3 is provided at a rear end of the operating shaft 12 of the treatment portion drive assembly 15. The connection portion 34 includes a coupling ball 35 which can be attached to or detached from the operating shaft coupling portion 19 of the movable handle 7. The coupling ball 35 can be engaged or disengaged with a lock groove (not shown) provided in the operating shaft coupling portion 19 of the movable handle 7 shown in FIG. 2C.

The forceps 1 according to the example is used in a state in which the sheath assembly 14, the treatment portion drive assembly 15 and the operating section assembly 16 are combined. When such a forceps 1 is assembled and used, a forceps cover sheath 45 (see FIG. 1A) which will be described below is attached. In other words, the forceps 1 is used with the forceps cover sheath 45.

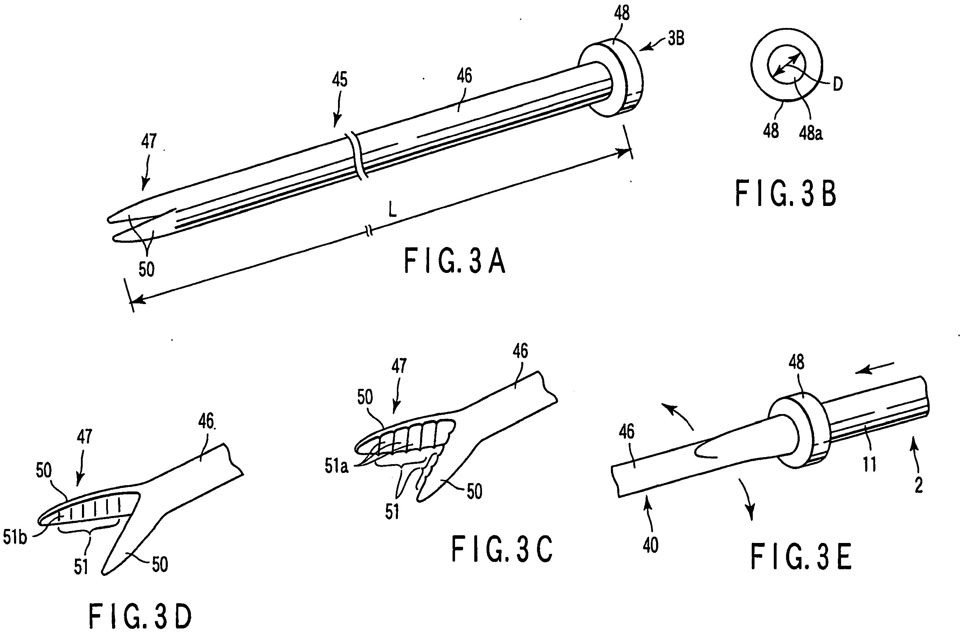

As shown in FIG. 3A, the forceps cover sheath 45 includes an elongated tube member (sheath main body) 46, a tip attachment portion 47 integrally formed at a distal end of the tube member 46, and a grab 48 integrally provided at a proximal end of the tube member 46. The tube member (cover member) 46 has elasticity which enables extension and retraction in its diametrical direction i.e. retractility elasticity. The tube member 46 is formed of, for example, polyurethane integrally with the tip attachment portion 47 as flexible thin resin materials.

As shown in FIG. 3B, the inside diameter of a proximal end portion of the tube member 46, that is, a forceps inlet 48a of the grab 48 is D. The inside diameter D is smaller than the outside diameter of the sheath 11 of the sheath assembly 14 shown in FIG. 2A. Because the inside diameter D of the tube member 46 has elasticity which extends and retracts the tube member 46 itself in the diametrical direction, the inside diameter D can change in accordance with the outside diameter of the sheath 11 of the sheath assembly 14.

An entire length L of the forceps cover sheath 45 shown in FIG. 3A is shorter than the length from the distal end portion of the treatment portion 10 of the forceps 1 to the distal end of the exterior 24 of the sheath 11 of the insertion section 2.

The tip attachment portion 47 of the forceps cover sheath 45 is formed slightly smaller than a shape corresponding to the treatment portion 10 of the forceps 1. In the example, since the treatment portion 10 includes the pair of jaws 30 that can relatively open and close, the tip attachment portion 47 of the forceps cover sheath 45 includes a jaw corresponding portion 50 which is formed to branch into two to correspond to the jaws 30 of the forceps 1.

Wall thickness of the tip attachment portion 47 of the forceps cover sheath 45 is thinly formed. Concavities and convexities corresponding to the teeth 30a, 30b are formed in the jaw corresponding portion 50 in accordance with the teeth 30a, 30b shown in FIG. 1B when the forceps cover sheath 45 is attached to the forceps 1. That is, when the forceps 1 is attached to the tip attachment portion 47, the concavities and convexities corresponding to the teeth 30a, 30b of the jaws 30 are formed in the tip attachment portion 47. The concavities and convexities have an anti-slip effect of tissues when the treatment target is gripped.

An anti-slip-treated portion (surface treated portion) 51 is formed on opposite surfaces of the jaw corresponding portions 50, as shown in FIG. 3C and FIG. 3D. As the anti-slip-treated portion 51, a tooth-shaped portion 51a corresponding to the teeth 30a, 30b (see FIG. 1B) of the jaws 30 is formed as shown in FIG. 3C. Alternatively, a satin finished portion 51b may be formed as shown in FIG. 3D.

A multiplier effect of the concavities and convexities which are produced by deformation of the jaw corresponding portions 50 corresponding to the teeth 30a, 30b, and the anti-slip-treated portion 51 prevents the treatment target from slipping on the anti-slip-treated portion 51. For example, the forceps cover sheath 45 is prevented from slipping on the treatment target when the anti-slip-treated portion 51 of the tip attachment portion 47 of the forceps cover sheath 45 contacts the treatment target or, for example, grips the treatment target.

As wall thickness of the tube member 46 and the tip attachment portion 47 of the forceps cover sheath 45 are thinly formed and have flexibility and elasticity, they deform in accordance with the shape of the treatment portion 10 of the forceps 1. Therefore, even in case of the deformation of the jaws 30 shown in FIG. 1B, the forceps cover sheath 45 can be used. In other words, in case that the jaws 30 shown in FIG. 1B has a shape curved in one direction, the cover sheath 45 deforms in accordance with the curved shape. The same forceps cover sheath 45 can be used even in a different type of equipment.

The grab 48 provided at the proximal end portion of the tube member 46 is ring-shaped. The inside diameter of the grab 48 is the same as the inside diameter D of the proximal end of the tube member 46. The grab 48 is thickly formed outwardly in the diametrical direction, and formed to be slightly more rigid than the tube member 46 for a user to easily hold the grab 48.

Next, function of the forceps 1 having the forceps cover sheath 45 will be described.

A method of attaching the forceps cover sheath 45 to the forceps 1 will be described.

As shown in FIG. 3E, when the cover sheath 45 is used, the treatment portion 10 of the forceps 1 is inserted into an inner space of the cover sheath 45 from the side of the grab 48 toward the tip attachment portion 47 through the tube member 46. As the tube member 46 of the cover sheath 45 is formed to have a diameter slightly smaller than the outside diameter of the insertion section 2 of the forceps 1, the cover sheath 45 is attached to the forceps 1 while closely contacting an outer periphery of the treatment portion 10 and the sheath 11 owing to its elasticity. In other words, the treatment portion 10 and the sheath 11 of the forceps 1 are inserted into the tube member 46 while increasing the diameter in the diametrical direction against a biasing force (elastic force) that prevents a diametrical increase of the tube member 46 of the cover sheath 45. Thus, the tube member 46 of the cover sheath 45 changes the shape in accordance with the shape of the treatment portion 10 and the sheath 11 of the forceps 1.

When the jaws 30 of the treatment portion 10 are disposed in the inner space of the tip attachment portion 47 of the treatment portion cover sheath 45, the operating section 3 (the handles 6, 7) is operated to open the jaws 30. Each of the jaws 30 is inserted into the forked jaw corresponding portion 50 of the tip attachment portion 47 of the cover sheath 45. At this time, the tube member 46 and the tip attachment portion 47 have the elasticity to extend and retract and the flexibility to be bent in accordance with the opening and closing of the jaws 30. Thus they maintain a state contacting the outer periphery of the treatment portion 10 of the forceps 1.

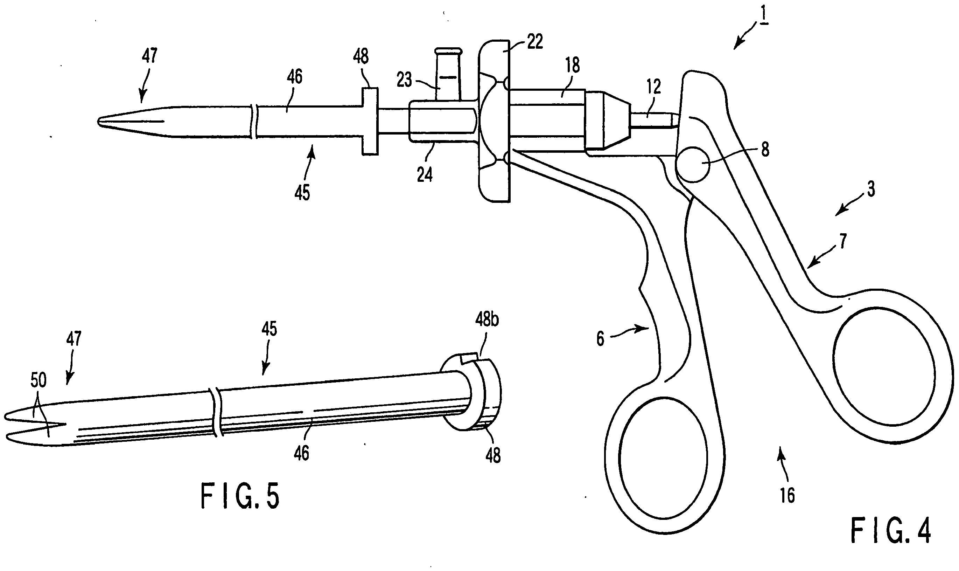

When the jaws 30 of the treatment portion 10 of the forceps 1 are disposed in the inner space of the tip attachment portion 47 of the cover sheath 45, the user stops further insertion of the forceps 1 into the cover sheath 45. The grab 48 of the cover sheath 45 is frictionally engaged with the outer periphery of the sheath 11 of the insertion section 2 of the forceps 1. At this time, since the original inside diameter D of the tube member 46 of the cover sheath 45 is smaller than the outside diameter of the insertion section 2 (sheath 11) of the forceps 1, the tube member 46 of the cover sheath 45 closely contacts the outside of the treatment portion 10 and the sheath 11 of the forceps 1 (see FIG. 4). In other words, the forceps cover sheath 45 is attached to the insertion section 2 of the forceps 1.

In the forceps 1 to which such a forceps cover sheath 45 is attached, operation of the operating section 3 also causes the jaws 30 of the treatment portion 10 to open and close without giving much resistance to the operation owing to the elasticity and flexibility of the cover sheath 45, thus treating the treatment target. In other words, the jaws 30 can be opened and closed with little force as the jaw corresponding portion 50 of the cover sheath 45 is formed to branch into two and has the flexibility to open and close pivotally on a proximal portion of the jaw corresponding portion 50. The tooth-shaped portion 51a formed in the jaw corresponding portion 50 of the tip attachment portion 47 of the cover sheath 45 prevents slipping when gripping the treatment target.

Next, a method of detaching the forceps cover sheath 45 from the forceps 1 will be described.

When the cover sheath 45 is detached from the forceps 1, for example, a syringe (not shown) is attached to the cleaning port 23. Water and air are conveyed from the syringe into the sheath 11 to inject a gas and a liquid between the forceps 1 and the cover sheath 45. The gas and liquid spout from a distal end opening of the sheath 11 of the sheath assembly 14. A space is formed by the gas and liquid between the cover sheath 45, and the treatment portion 10 and the insertion section 2 of the forceps 1. This reduces a frictional force between the tube member 46 of the cover sheath 45, and the treatment portion 10 and the sheath 11 of the forceps 1. This releases the tube member 46 of the cover sheath 45, and the treatment portion 10 and the sheath 11 of the forceps 1 from the close contact state to allow easy detachment. The detached cover sheath 45 is discarded.

The forceps 1 from which the forceps cover sheath 45 is detached has not itself directly touched the treatment target at a part fitted with the cover sheath 45. Thus, the part fitted with the cover sheath 45 is not stained due to the treatment target. In other words, a distal end portion side of the forceps 1, in particular, of the treatment portion 10 and the sheath 11 is the region which is fitted with the cover sheath 45 so that it is not easily stained. Therefore, that extremely reduces to spend time for cleaning process for the forceps 1. As the link mechanism 9 can especially be prevented from being stained, labor and time to elaborately clean particularly the link mechanism 9 can be reduced.

As described above, the following effects can be obtained according to the example.

In case that the forceps cover sheath 45 covers at least the treatment portion 10 of the forceps 1, blood and body fluids of the treatment target can be prevented from sticking to the link mechanism 9 of the treatment portion 10 when the forceps 1 is used. In other words, by using the forceps 1 in such a manner that the cover sheath 45 is attached to the treatment portion 10 and a distal end portion side of the sheath 11 of the forceps 1, the treatment portion 10 and the link mechanism 9 can be prevented from being stained with, for example, the blood and body fluids from the treatment target. Thereby, it is possible to significantly reduce the labor and time to perform the cleaning process for treatment portion 10 and the inside (link mechanism 9) of the sheath 11 the forceps 1.

The forceps cover sheath 45 changes its shape in accordance with the end shape of the treatment portion 10 of the forceps 1. Thus, the cover sheath 45 can be used for forcipes having various shapes of the treatment portion 10. In case that the shape of the distal end portion of the cover sheath 45 is properly selected to operate the operating section 3 of the forceps 1, the treatment portion 10 can be easily operated to perform a treatment.

The tip attachment portion 47 of the forceps cover sheath 45 may be formed slightly larger than the shape that corresponds to the treatment portion 10 of the forceps 1. When the tip attachment portion 47 of the cover sheath 45 is formed slightly smaller than the shape that corresponds to the treatment portion 10 of the forceps 1, it fits more tightly and can be prevented from being displaced from the jaws 30 of the forceps 1.

When the forceps cover sheath 45 is detached from the forceps 1, the cover sheath 45 may be turned over and rolled up from the grab 48 toward the tip attachment portion 47 without conveying air and water from the cleaning port 23. Further, as shown in FIG. 5, if, for example, a notch 48b is formed in the grab 48, the cover sheath 45 can be removed by cutting from the notch 48b. The notch 48b is formed, for example, as a V-groove from the outer periphery to the center of the grab 48. Such a notch 48b may be formed to the vicinity of the outer periphery of the proximal end portion of the tube member 46 of the forceps cover sheath 45.

In the configuration of the example described above, the grab 48 is provided at the proximal end portion of the forceps cover sheath 45. As the forceps cover sheath 45 closely contacts of the treatment portion 10 and the sheath 11 of the forceps 1 to be in a frictional engagement, the grab 48 may be omitted and the proximal end portion of the tube member 46 of the forceps cover sheath 45 may be wound with, for example, a surgical tape (not shown) to attach and fix the cover sheath 45 to the sheath 11.

When the entire length L of the forceps cover sheath 45 is long enough to protect the link mechanism 9 of the forceps 1 and the proximal end portion of the forceps cover sheath 45 has a length to be located in a body cavity, the grab 48 does not need to be provided.

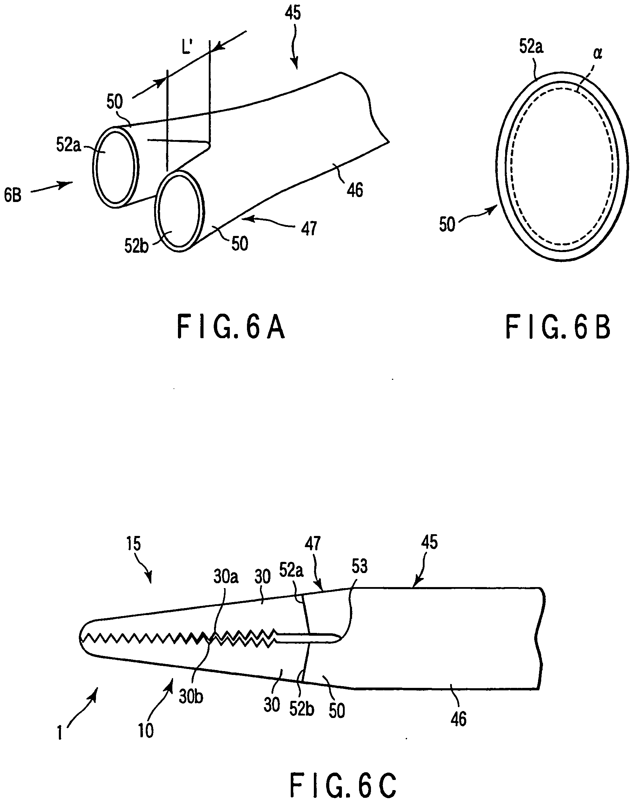

A forceps cover sheath 45 to be used with the surgical forceps according to the present invention will be described by use of FIG. 6A to FIG. 6C.

It has been described that the jaw corresponding portion 50 of the tip attachment portion 47 of the cover sheath 45 described above is closed. Here, as shown in FIG. 6A, the jaw corresponding portion 50 of the tip attachment portion 47 includes openings 52a, 52b at the distal end. In this case, a distance L' from the openings 52a, 52b to a forked top portion 53 of the jaw corresponding portion 50 is formed shorter than the entire length of the jaws 30.

As shown in FIG. 6B, an internal peripheral length α of the openings 52a, 52b is formed slightly smaller than an outer peripheral length of the section of the jaws 30 of the forceps 1 to which the openings 52a, 52b correspond respectively when the cover sheath 45 is attached to the forceps 1. Since the cover sheath 45 expands and contracts, the openings 52a, 52b closely contact the jaws 30 of the forceps 1.

As shown in FIG. 6C, when the jaw corresponding portion 50 has the openings 52a, 52b, the cover sheath 45 is attached to the forceps 1 with the same function as described above. In this case, the forked top portion 53 of the cover sheath 45 is located in the vicinity of the link mechanism 9 of the jaws 30. As the distance L' from the openings 52a, 52b to the forked top portion 53 is shorter than the entire length of the jaws 30, the distal end portion of the jaws 30 is exposed outside. Thus, the teeth 30a, 30b of the jaws 30 are also exposed outside. As the internal peripheral length α of the openings 52a, 52b is shorter than the outer peripheral length of the section of the jaws 30, the internal peripheral surfaces of the openings 52a, 52b closely contact the outer peripheral surface of the jaws 30.

Thus, the teeth 30a, 30b of the jaws 30 are exposed outside even when the jaw corresponding portion 50 includes the openings 52a, 52b, so that gripping performance inherent in the forceps 1 can be maintained and the gripping performance can be prevented from being impaired. The openings 52a, 52b closely contact the jaws 30, so that the link mechanism 9 can prevent from being stained.

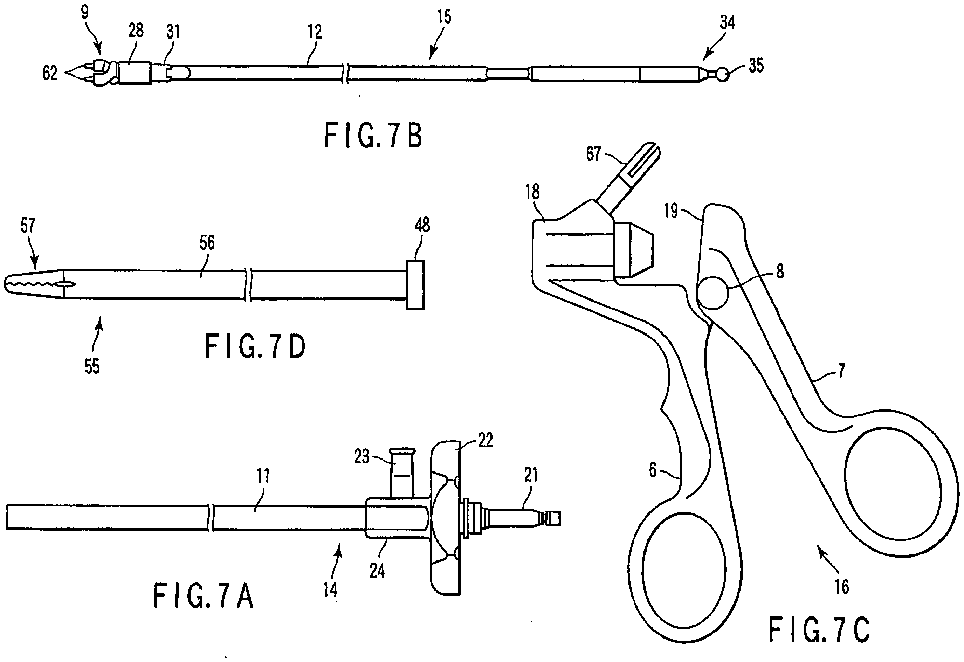

Next, a second example will be described referring to FIG. 7A to FIG. 11C. The example is a modification of the first example, the same members as those described in the first example are denoted with the same reference numerals, and detailed description is omitted.

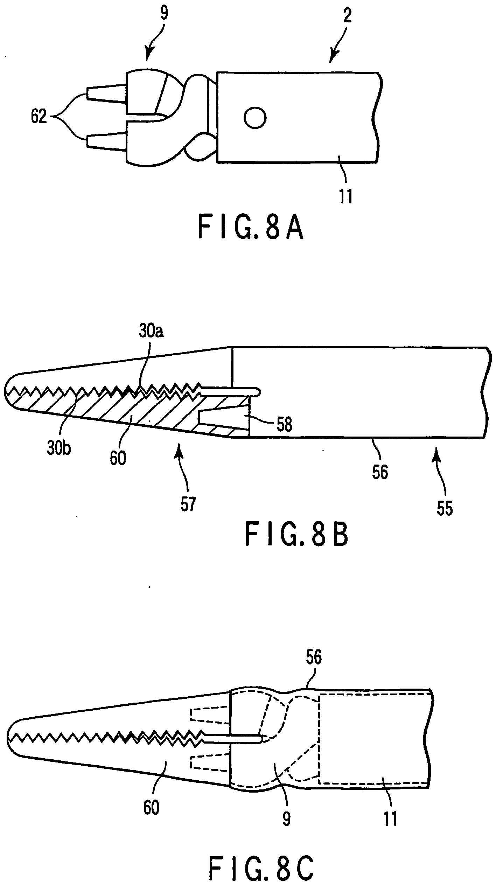

The sheath assembly 14 shown in FIG. 7A has the same configuration as that of the sheath assembly 14 shown in FIG. 2A. As shown in FIG. 7D and FIG. 8B, a forceps cover sheath 55 according to the example includes a treatment portion component 57 at the distal end of a tube member (sheath main body) 56. The treatment portion component 57 is integrally provided (connected) at the distal end of the tube member 56 by, for example, welding or adhesive bonding to close the distal end of the tube member 56.

The treatment portion component 57 is preferably formed of a conductive member. The tube member 56 has the elasticity to enable extension and retraction in its diametrical direction, and is formed of, for example, polyurethane as flexible thin resin materials. As the tube member 56 itself has the elasticity to extend and retract in the diametrical direction, the inside diameter D of the tube member 56 can be changed with the outside diameter of the sheath 11 of the sheath assembly 14.

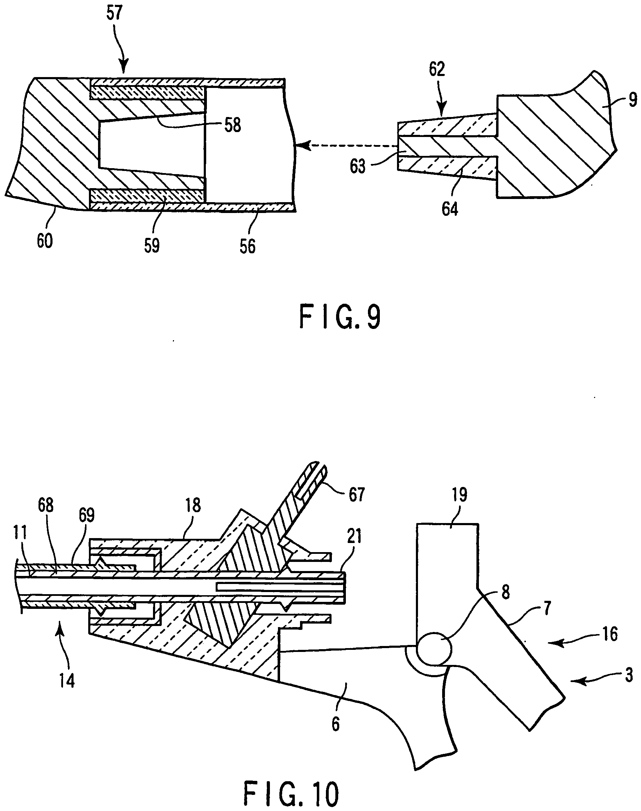

As shown in FIG. 8B and FIG. 9, a tapered fitting concavity 58 into which a fitting convexity 62 described later is to be fitted is provided at the proximal end portion of the treatment portion component 57. As shown in FIG. 8B, a pair of jaws (forceps teeth) 60 having the teeth 30a, 30b on the opposite surfaces is provided in the treatment portion component 57 which is provided with the fitting concavity 58.

As shown in FIG. 9, a heat-insulating member 59 is disposed on the whole outer periphery of the proximal end portion of the jaws 60. The insulating member 59 is preferably provided at least from the proximal end of the jaws 60 to correspond to the depth of the fitting concavity 58. The outer periphery of the insulating member 59 is covered with the tube member 56 by an adhesive (not shown) which bonds the tube member 56 to the treatment portion component 57. The outer periphery of the proximal end portion of the jaws 60 is covered with the insulating member 59 to prevent heat conducted from a conductive terminal 63 described later from transmitting to the tube member 56 to damage the tube member 56.

On the other hand, as shown in FIG. 7B, FIG. 8A and FIG. 9, the link mechanism 9 of the treatment portion drive assembly 15 is provided with the protruding fitting convexities 62 instead of the jaws 30 (see FIG. 2B) described in the first example. The fitting convexity 62 includes the conductive terminal 63 formed, for example, integrally with the link mechanism 9, and a resin portion 64 which is disposed substantially in a truncated cone shape around the conductive terminal 63 and which is formed of resin materials such as silicone.

Thus, the fitting convexity 62 has conductivity due to the conductive terminal 63. The resin portion 64 is tapered, and engaged and fixed by frictional engagement when fitted into the fitting concavity 58. The frictional force between the fitting concavity 58 and the fitting convexity 62 can be kept high by the resin portion 64, thereby maintaining a favorable fitting state (fixing force).

After the fitting convexity 62 is repeatedly fitted into the fitting concavity 58 of the treatment portion component 57 having the distal end portion of a desired shape, the resin portion 64 of the fitting convexity 62 is abraded away. This gradually decreases the frictional force (fixing force) between the fitting concavity 58 and the fitting convexity 62, so that it is possible to easily know when to replace the resin portion 64 of the link mechanism 9 of the forceps 1. It is thus possible to easily determine the commutative moment i.e. duration of life of the forceps 1 itself on the basis of an amount of the abraded resin portion 64 of the fitting convexity 62.

The operating section assembly 16 shown in FIG. 7C and FIG. 10 is formed as follows.

The fixed handle 6 and the movable handle 7 of the operating section assembly 16 are made of electrical insulating materials such as plastic materials. In an alternative configuration, the surface of the conductive materials such as a metal of the fixed handle 6 and the movable handle 7 may be covered with electrical insulating materials.

A high frequency code (HF code) connection pin 67 is insert-molded in the sheath coupling member 18 of the fixed handle 6 of the forceps 1 according to the example. The HF code connection pin 67 is formed of conductive materials. An inner end portion of the HF code connection pin 67 is connected to the handle coupling portion 21 of the sheath assembly 14. An unshown HF code is connected to an outer end portion of the HF code connection pin 67. A power supply device (an energy supplying device) (not shown) to supply a high frequency current to the HF code is connected to the HF code.

To the sheath 11 of the sheath assembly 14, an outer pipe 69 which is formed of heat-resistant insulating materials such as plastic materials is attached on an outer peripheral surface of an inner pipe 68 made of conductive materials such as a metal. The handle coupling portion 21 of the sheath assembly 14 is formed of conductive materials such as a metal, and is connected to the inner pipe 68 of the sheath 11. The operating shaft 12 of the treatment portion drive assembly 15 is electrically connected to the inner pipe 68. The operating shaft 12 is electrically connected to the link mechanism 9 described above.

Next, function of such a forceps 1 will be described. A method of attaching the forceps cover sheath 55 to the forceps 1 will be described.

The link mechanism 9 of the forceps 1 is inserted from the proximal end to distal end of the tube member 56 of the forceps cover sheath 55. The fitting convexity 62 at the distal end of the link mechanism 9 is located at a position opposite to the fitting concavity 58 of the jaws 60 of the forceps cover sheath 55. The fitting convexities 62 are inserted into the fitting concavities 58. The respective tapered surfaces of the fitting concavity 58 and the fitting convexity 62 are frictionally engaged and fixed to each other. The conductive terminal 63 of the fitting convexity 62 is electrically connected to the fitting concavity 58. In other words, the link mechanism 9 of the forceps 1 and the treatment portion component 57 of the forceps cover sheath 55 are electrically connected to each other and held so that the treatment portion component 57 can move in conjunction with the link mechanism 9 (see FIG. 8C).

In this way, the jaws 60 of the treatment portion component 57 in the embodiment operate in the same manner as the pair of jaws 30 of the treatment portion 10 described in the first example. Thus, in case that the operating section 3 is operated, the treatment portion component 57 of the forceps cover sheath 55 operates in conjunction with the link mechanism 9.

When the link mechanism 9 of the forceps 1 is attached to the treatment portion component 57 of the forceps cover sheath 55, the user stops further insertion of the forceps 1 into the forceps cover sheath 55. The grab 48 of the forceps cover sheath 55 is frictionally engaged with the outer periphery of the sheath 11 of the insertion section 2 of the forceps 1. At this time, since the original inside diameter D of the tube member 56 of the forceps cover.sheath 55 is smaller than the outside diameter of the insertion section 2 (sheath 11) of the forceps 1, the tube member 56 of the forceps cover sheath 55 closely contacts the outside of the treatment portion 10 and the sheath 11 of the forceps 1.

When a high frequency current is passed from the above-described power supply device to the HF code connection pin 67 by the HF code, the high frequency current is transmitted from the operating shaft 12 and the link mechanism 9 of the forceps 1 to the treatment portion component 57 of the forceps cover sheath 55. Therefore, the treatment target is subjected to a desired high frequency treatment at the jaws 60 of the treatment portion component 57. The tube member 56 is prevented from being damaged by heat generated by the high frequency current as the tube member 56 is disposed on the outer periphery of the insulating member 59.

Next, a method of detaching the forceps cover sheath 55 from the forceps 1 will be described.

When the forceps cover sheath 55 is detached from the forceps 1, for example, the syringe (not shown) is attached to the cleaning port 23. Water and air are conveyed from the syringe into the sheath 11 to inject a gas and a liquid between the forceps 1 and the forceps cover sheath 55.

The gas and liquid spout from the end opening of the sheath 11 of the sheath assembly 14, and a space is formed by the gas and liquid between the forceps cover sheath 55 and the insertion section 2 (sheath 11) of the forceps 1. This reduces a frictional force between the tube member 56 of the forceps cover sheath 55 and the insertion section 2 (sheath 11) of the forceps 1. This releases the tube member 56 of the forceps cover sheath 55 and the insertion section 2 of the forceps 1 from the close contact state.

The fitting of the fitting concavity 58 of the treatment portion component 57 and the fitting convexity 62 at the end of the link mechanism 9 is canceled. The forceps cover sheath 55 is easily detached from the forceps 1.

Subsequently, the used forceps cover sheath 55 is discarded.

As described above, the following effects can be obtained according to the example.

Because the treatment portion component 57 is detachable from the forceps 1, the treatment portion component 57 can be properly selected in accordance with the procedure applied to the treatment target. Therefore, various kinds of treatment portion components 57 can be used which, for example, have not only the jaws 60 shown in FIG. 8B but also the jaws 60 with a curved tip end.

By forming the treatment portion component 57 with conductive materials and leading high frequency energy from the side of the operating section 3, a high frequency treatment can be performed using the treatment portion component 57 as an electric scalpel.

In case that the resin portion 64 covering the conductive terminal 63 of the fitting convexity 62 is frictionally fitted into the fitting concavity 58, it is possible to easily ascertain how the resin portion 64 is fitted into the fitting concavity 58. In other words, it is possible to recognize how much the forceps 1 has been used from frictional reduction of the resin portion 64 of the fitting convexity 62.

In the configuration of the example described above, the fitting convexity 62 is provided at the distal end portion of the link mechanism 9 and the fitting concavity 58 is provided at the proximal end portion of the treatment portion component 57, but it is also preferable that the fitting convexity 62 and the fitting concavity 58 may be provided in the other way around.

It has mainly described in the example that the conductive member is used to pass the high frequency current through the treatment portion component 57, but naturally, nonconductive materials may also be used.

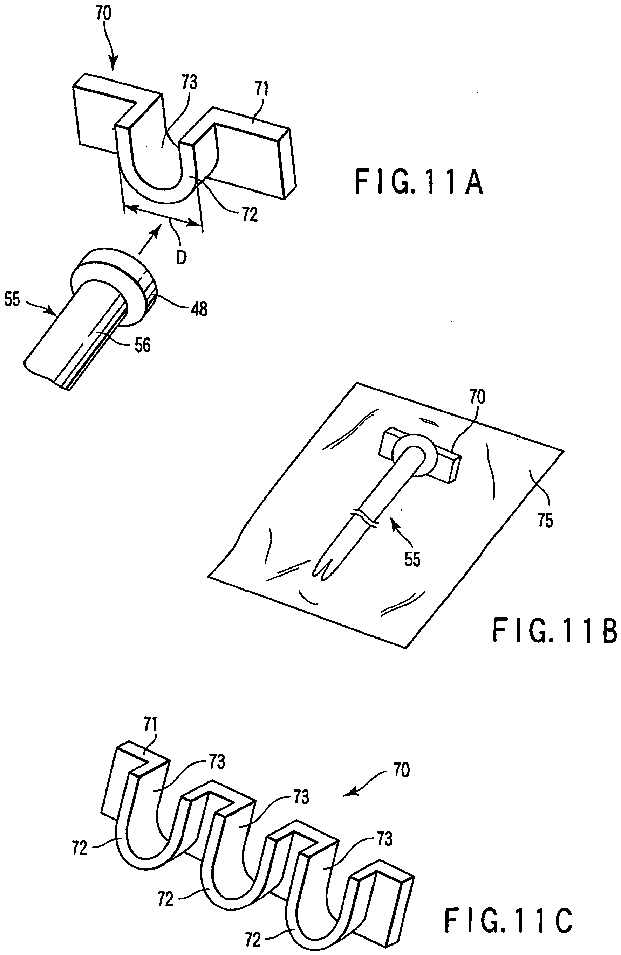

In performing an operation, for example, an attachment aid 70 shown in FIG. 11A is preferably used when the forceps cover sheath 55 is attached to the forceps 1. As shown in FIG. 11A, the attachment aid 70 includes an aid main body 71, a U-shape protrusion 72 provided in the aid main body 71, and a notch 73 which is provided in the protrusion 72 and which penetrates the aid main body 71. The maximum outside diameter of the protrusion 72 is formed larger than the inside diameter D of the proximal end portion of the forceps cover sheath 55 shown in FIG. 3B. The grab 48 is elastically deformed to be attached to the protrusion 72.

In a state where the forceps cover sheath 55 is attached to the attachment aid 70 by use of the grab 48, the forceps 1 is inserted from the notch 73. The attachment aid 70 secures a space on a rear end side of the forceps cover sheath 55 so that the treatment portion 10 of the forceps 1 can be easily inserted into the inner space of the tube member 46.

When the forceps cover sheath 55 is to be detached from the attachment aid 70, the forceps cover sheath 55 can be detached from the attachment aid 70 with a slight force. Thus, the forceps cover sheath 55 is attached to the forceps 1 and detached from the attachment aid 70 when the forceps 1 is used.

As shown in FIG. 11B, the forceps cover sheath 55 is preferably stored in a peel bag 75 in a state attached to the protrusion 72 of the attachment aid 70 and in a sterile state. In this way, the forceps cover sheath 55 attached to the attachment aid 70 can be produced from the peel bag 75 and used in a sterilized state every time it is used.

As shown in FIG. 11C, for example, the three protrusions 72 are provided in the aid main body 71 of the attachment aid 70. In performing an operation, a plurality of forcipes 1 of the same kind or different kinds may be used as the forceps cover sheath 55 is stained or as different treatments are performed. In this case, the attachment aid 70 having, for example, the three protrusions 72 is preferably used.

When the forceps 1 is used in an operation, it is necessary to attach the forceps cover sheath 55 to the forceps 1 to be used. In the attachment aid 70 shown in FIG. 11C, for example, the forceps cover sheaths 55 (three sheaths in FIG. 11C) to be used in one case are combined into one package, and the forceps cover sheaths 55 with desired shapes are attached to the attachment aid 70 in order of the forcipes 1 that are expected to be used. It is possible to save labor to open the peel bag 75 of the forceps cover sheath 55 during an operation every time the forceps cover sheath 55 is attached to the forceps 1.

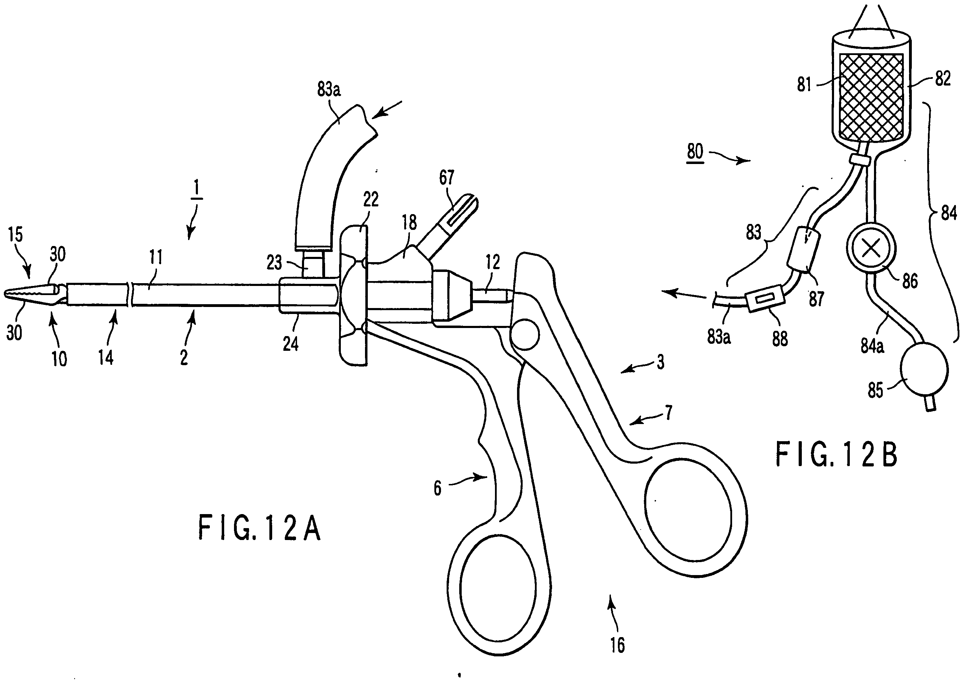

Next, a third example will be described referring to FIG. 12A to FIG. 13B. The example is a modification of the first and second examples, the same members as those described in the first example are denoted with the same reference numerals, and detailed description is omitted.

A water conveyance system 80 shown in FIG. 12B is connected to the cleaning port 23 shown in FIG. 12A. The water conveyance system 80 includes a physiological saline package 81, a package storing portion 82 which stores the physiological saline package 81, and an infusion tube section 83 connected to the physiological saline package 81. The infusion tube section 83 infuses a liquid supplied from the physiological saline package 81 into the port 23. The package storing portion 82 includes a pressure section 84 which controls pressure applied to the physiological saline package 81.

The pressure section 84 includes a balloon 85 which is held by an operator to bring pressure through a tube 84a, and a pressure gauge 86 which measures the pressure in the tube 84a. That is, the pressure gauge 86 can be used to know the pressure applied to the physiological saline package 81. The infusion tube section 83 includes a liquid volume observation port 87 connected to the physiological saline package 81 through a tube 83a, and a liquid volume adjustment portion 88 which adjusts flow volume of physiological saline flowing in the tube 83a. An end of the tube 83a is connected to the port 23 of the forceps 1. The physiological saline can thus be supplied from the physiological saline package 81 to the cleaning port 23 of the forceps 1 through the infusion tube section 83.

The package storing portion 82 includes, inside a holding portion 89 which is, for example, meshed, a balloon 90 which is pressurized by the pressure section 84, and a space portion 91 in which the physiological saline package 81 is disposed.

Next, the function of the forceps 1 according to the example will be described.

In the case that the balloon 85 of the pressure section 84 is held while disposing the physiological saline package 81 in the space portion 91, the balloon 90 of the package storing portion 82 is expanded through the tube 84a. The balloon 90 then presses the physiological saline package 81 disposed in the space portion 91, and due to the pressure thus produced, the physiological saline runs out toward the liquid volume observation port 87 through the tube 83a. The physiological saline which has run out is one retained at the liquid volume observation port 87, and its flow volume is adjusted by the liquid volume adjustment portion 88. That is, a desired amount (e.g., drop by drop at predetermined time intervals) of physiological saline is introduced from the physiological saline package 81 into the port 23 through the infusion tube 83a.

The physiological saline is led out from a distal end portion of the sheath assembly 14 which communicates with the port 23. Just before the physiological saline is led out, a physiological saline is poured on the link mechanism 9. The link mechanism 9 is prevented from being stained. That is, while the physiological saline poured on the link mechanism 9 is removing the stains on the link mechanism 9, the physiological saline is introduced from the distal end portion of the sheath assembly 14. Therefore, stains to stick or sticking on the link mechanism 9 are washed away constantly or at times while the forceps 1 is being used.

In this way, the link mechanism 9 is not easily stained during an operation, so that the labor and time of the cleaning after the forceps 1 is used, can be significantly reduced.

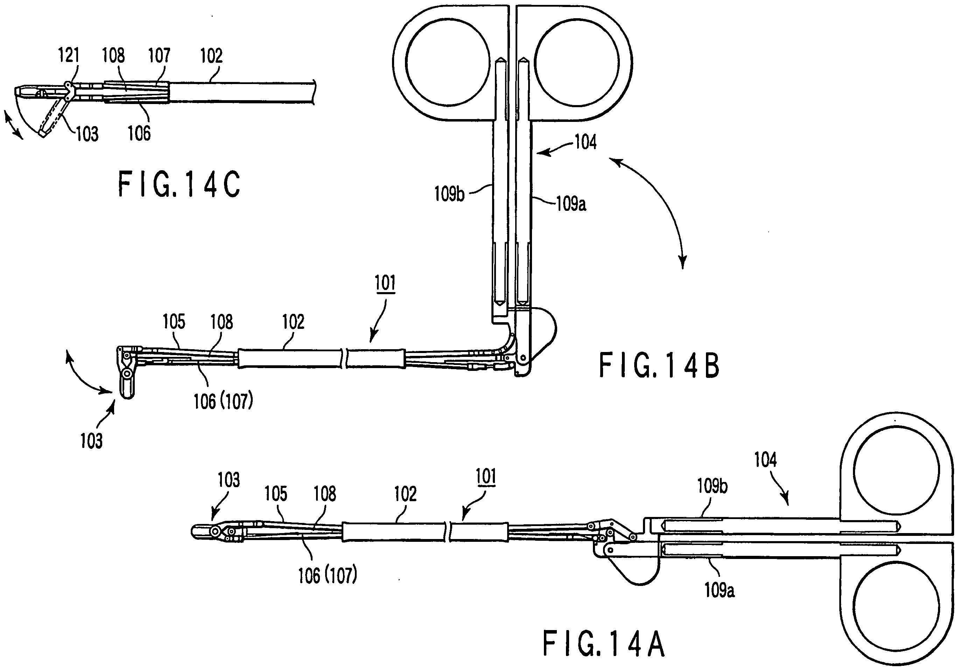

Next, a fourth example will be described referring to FIG. 14A to FIG. 15. The example is a modification of the first example, the same members as those described in the first example are denoted with the same reference numerals, and detailed description is omitted.

FIG. 14A to FIG. 14C show a surgical forceps 101 which is a modification of the forceps 1 (see FIG. 1) described in the first example. As shown in FIG. 14A to FIG. 14C, the forceps 101 includes an elongated insertion section 102. At a distal end portion of the insertion section 102, a treatment portion 103 having a link mechanism which is more complex than that described in the first example is disposed. At a proximal end of the insertion section 102, an operating section 104 is provided to operate the link mechanism and to open/close and pivot the treatment portion 103.

In the insertion section 102, for example, first to third drive rods 105, 106 and 107 are provided in parallel. A support portion 108 of the insertion section 102 is provided between the first drive rod 105 and the second and third drive rods 106 and 107, preventing mutual interference among the drive rods 105, 106 and 107.

The operating section 104 includes operating handles 109a and 109b which pivot and open/close with respect to an axial direction of the insertion section 102. Proximal ends of the three drive rods 105, 106 and 107 are coupled to distal ends of the operating handles 109a and 109b by the link mechanism. The operating section 104 pivots from a state straight in the axial direction of the insertion section 102 (see FIG. 14A) to a state orthogonal in a predetermined plane (see FIG. 14B). The operation section 104 also pivots within predetermined angles in a direction orthogonal to that plane.

Next, the treatment portion 103 (mainly the link mechanism) will be described.

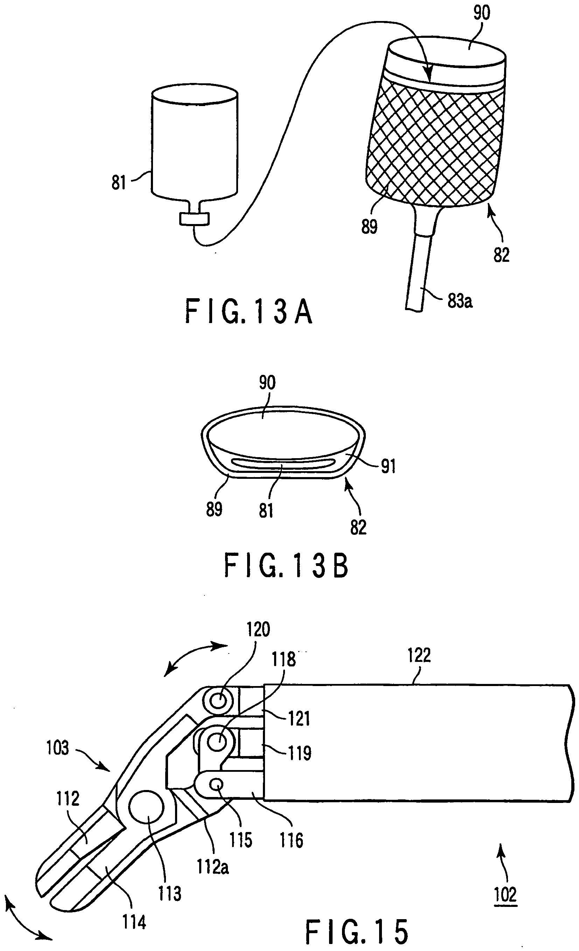

As shown in FIG. 15, a pair of jaws 112, 114 is coupled to the treatment portion 103 of the insertion section 102 by a pivot support pin 113 so that they can relatively open and close. A bending portion 112a is provided at a proximal end portion of the first jaw 112. A first coupling member 116 is coupled to a proximal end portion of the bending portion 112a by a first coupling pin 115 having a lateral axis. A proximal end side of the first coupling member 116 is laterally wide. Second and third coupling pins (not shown) respectively having axes in a vertical direction are spaced apart laterally from each other at the proximal end side of the first coupling member 116 (see FIG. 14C).

The second coupling pin is coupled to the second drive rod 106, and the third coupling pin is coupled to the third drive rod 107. The first jaw 112 pivots laterally and vertically on the vertical and lateral pins. A first pivot support pin 118 having a lateral axis is provided at a proximal end portion of the first jaw 112 (bending portion 112a). A pivot plate 119 is coupled to the first pivot support pin 118. The pivot plate 119 is coupled with a vertical axis at a distal end portion of the support portion 108 and pivots in a lateral direction.

One end of a second coupling member 121 is pivotally coupled to a proximal end portion of the second jaw 114 by a second coupling pin 120 having a lateral axis. A third coupling member is coupled to the other end of the second coupling member 121 by a pivot support pin (not shown) having a vertical axis. The other end of the third coupling member is pivotally coupled to a distal end portion of the first drive rod 105 by a fourth coupling pin (not shown) having a lateral axis. The second jaw 114 pivots laterally and vertically on the vertical and lateral pins. That is, the first and second jaws 112 and 114 pivot vertically and laterally.

Therefore, the jaws 112 and 114 of the treatment portion 103 of the forceps 101 not only open/close pivotally on the pivot support pin 113 but also pivot laterally and vertically. That is, the forceps 101 includes the link mechanism which is much more complex than that of the forceps 1 in the first example. The insertion section 102 of the forceps 101 is covered with a sheath 122.

As the treatment portion 103 having such a complex link mechanism is provided at a distal end portion of the insertion section 102, stains such as blood and body fluids of a patient might be brought to the various link mechanism when the treatment portion 103 is cleaned. Therefore, particularly elaborate cleaning needs to be performed to ensure that the stains are removed. In order to reduce such labor and time to clean the forceps 101, the forceps cover sheath 45 is used to cover the treatment portion 103 and the link mechanism of the forceps 101.

The forceps cover sheath 45 is formed, for example, in the same manner as the cover sheath 45 described in the first example. A distal end portion of the forceps cover sheath 45 is formed to have a shape similar to the shape of the treatment portion 103 of the forceps 101 according to the example. The entire treatment portion 103 and a desired length of the sheath 122 which covers the insertion section 102 are covered with such a forceps cover sheath 45 rearward from their distal ends.

The tube member 46 described above is flexible, so that even if the treatment portion 103 pivots vertically and laterally at the distal end of the link mechanism, the tube member 46 pivots accordingly. Even if the jaws 112 and 114 are opened/closed while the treatment portion 103 of the forceps 101 pivots in a desired direction, a distal end portion of the tube member 46 pivots in accordance with their opening/closing.

After the treatment target is treated with the forceps 101 according to the example, the forceps cover sheath 45 is detached from the forceps 101 as described in the first example.

The forceps cover sheath 45 prevents the treatment portion 103 of the forceps 101 from being stained with the blood and body fluids from the patient. Therefore, even in the case of the treatment portion 103 including the complex link mechanism as described in the example, the labor and time to clean the link mechanism (the treatment portion 103) can be reduced. As shown in FIG. 14A to FIG. 14C, the treatment portion 103 having the link mechanism which is more complex than that described in the first example is disposed at the distal end of the forceps 101. The treatment portion 103 of the forceps 101 can pivot vertically and laterally with respect to the insertion section 102, and relatively opens/closes the pair of jaws (treatment pieces) 112 and 114.

As described above, the following effects can be obtained according to the example.

Even in the case of the significantly complex link mechanism having treatment portion 103 which includes a plurality of links, pivots vertically and laterally and opens/closes the jaws, a normal treatment can be performed while the complex link mechanism is covered to prevent staining. Therefore, the link mechanism is not easily stained, and the labor and time can be significantly reduced to clean the forceps 101, and moreover, the forceps 101 can be reused through the cleaning, disinfecting and sterilizing processes. Thus, the forceps 101 does not need to be disposed of, and costs of expensive medical equipment (forceps) having the complex link mechanism can be reduced. When the forceps 101 is cleaned, cleaning time can be reduced because the forceps 101 is not heavily stained originally.

Additional advantages and modifications will readily occur to those skilled in the art. Therefore, the invention in its broader aspects is not limited to the specific details and representative embodiments shown and described herein. Accordingly, various modifications may be made.

## Claims

1. A surgical forceps (1) comprising:
an elongated insertion section (2) having a distal and proximal end portions;
a link mechanism (9) provided at the distal end portion of the insertion section;
a treatment portion (10) which is coupled to the link mechanism and movable by operation of the link mechanism (9), wherein the treatment portion (10) comprises a pair of jaws (30):
an operating section (3) which is provided at the proximal end portion of the insertion section and which is adapted to move the link mechanism by remote control; and
a cover sheath (45) comprising:
a cover sheath main body (46) provided detachably from the forceps (1) and adapted to cover at least the link mechanism (9) out of the forceps (1),
**characterized in that** the cover sheath main body (46) comprises a tip attachment portion (47) at a distal end thereof, wherein the tip attachment portion (47) comprises:
a branched portion (53) branched into two and adapted to cover proximal end portions of the pair of jaws (30) of the treatment portion (10), and
a jaw corresponding portion (50) extending from the branched portion (53) and adapted to cover at least proximal end portions of the jaws (30) of the treatment portion (10), wherein the jaw corresponding portion (50) comprises:
at least two openings (52a, 52b) having internal peripheral surfaces adapted to be in close contact with outer peripheral surfaces of the jaws (30), wherein an internal peripheral length of the openings (52a, 52b) is formed shorter than an outer peripheral length of the jaws (30).

2. The surgical forceps (1) according to claim 1, **characterized in that** the cover sheath main body (46) has flexibility and elasticity.

3. The surgical forceps (1) according to claims 1 or 2, **characterized in that** the cover sheath main body (46) is formed of resin materials.

4. The surgical forceps (1) according to claim 3, **characterized in that** the resin materials are polyurethane.

5. The surgical forceps (1) according to claim 1, wherein a distance from the branched portion (53) to the openings is formed shorter than entire length of the jaws (30).

6. The surgical forceps (1) according to claim 1, **characterized in that** the cover sheath main body (46) includes a ring-shaped grab (48) at a proximal end thereof which is adapted to be held by an operator and to frictionally engage the elongated insertion section.

7. The surgical forceps (1) according to one of claims 1 to 6, **characterized in that** the insertion section (2) includes a sheath and a shaft, the sheath (11) having distal and proximal end portions which are adapted to cover the shaft of the insertion section, and the sheath includes a port (23) at the proximal end portion adapted to convey air and water toward the link mechanism (9) through an inner space of the sheath.

8. The surgical forceps system according to one of claims 1 or 7, wherein the cover sheath main body (46) is adapted to change shape in accordance with shapes of the insertion section (2), the treatment portion (10) and the link mechanism (9), and the cover sheath main body (46) is attachable to and detachable from an outer periphery of the link mechanism (9).

## Patentansprüche

1. Chirurgische Zange (1) mit:
einem länglichen Einführabschnitt (2) mit einem distalen und proximalen Endabschnitt;
einem Verbindungsmechanismus (9), der an dem distalen Endabschnitt des Einführabschnitts vorgesehen ist;
einem Behandlungsabschnitt (10), der mit dem Verbindungsmechanismus gekoppelt ist und durch Betätigen des Verbindungsmechanismus (9) bewegbar ist, wobei der Behandlungsabschnitt (10) ein Paar Greifbacken (30) aufweist;
einem Betätigungsabschnitt (3), der an dem proximalen Endabschnitt des Einführabschnitts vorgesehen ist, und der den Verbindungsmechanismus durch Fernsteuerung zu bewegen vermag; und
einer Abdeckhülle (45) mit:
einem Abdeckhüllen-Hauptkörper (46), der von der Zange (1) abnehmbar vorgesehen ist und zumindest den Verbindungsmechanismus (9) der Zange (1) abzudecken vermag,
**dadurch gekennzeichnet, dass** der Abdeckhüllen-Hauptkörper (46) einen Spitzen-Befestigungsabschnitt (47) an seinem distalen Ende aufweist, wobei der Spitzen-Befestigungsabschnitt (47) umfasst:
einen Verzweigungsabschnitt (53), der in zwei Zweige verzweigt ist und proximale Endabschnitte des Paars Greifbacken (30) des Behandlungsabschnitts (10) abzudecken vermag, und
einen den Greifbacken korrespondierenden Abschnitt (50), der sich von dem Verzweigungsabschnitt (53) erstreckt und zumindest proximale Endabschnitte der Greifbacken (30) des Behandlungsabschnitts (10) abzudecken vermag, wobei der den Greifbacken korrespondierende Abschnitt (50) umfasst:
mindestens zwei Öffnungen (52a, 52b) mit Innenumfangsflächen , die in engen Kontakt mit Außenumfangsflächen der Greifbacken (30) treten können, wobei eine Innenumfangslänge der Öffnungen (52a, 52b) kürzer ausgebildet ist als eine Außenumfangslänge der Greifbacken (30).

2. Chirurgische Zange (1) nach Anspruch 1, **dadurch gekennzeichnet, dass** der Abdeckhüllen-Hauptkörper (46) Flexibilität und Elastizität aufweist.

3. Chirurgische Zange (1) nach Anspruch 1 oder 2, **dadurch gekennzeichnet, dass** der Abdeckhüllen-Hauptkörper (46) aus Harzmaterialien gebildet ist.

4. Chirurgische Zange (1) nach Anspruch 3, **dadurch gekennzeichnet, dass** die Harzmaterialien Polyurethan sind.

5. Chirurgische Zange (1) nach Anspruch 1, wobei ein Abstand von dem Verzweigungsabschnitt (53) zu den Öffnungen kürzer gestaltet ist als die Gesamtlänge der Greifbacken (30).

6. Chirurgische Zange (1) nach Anspruch 1, **dadurch gekennzeichnet, dass** der Abdeckhüllen-Hauptkörper (46) eine ringförmige Klaue (48) an seinem proximalen Endabschnitt aufweist, die von einer Betätigungsperson gehalten zu werden vermag und mit dem länglichen Einführabschnitt in Reibungseingriff zu kommen vermag.

7. Chirurgische Zange (1) nach einem der Ansprüche 1 bis 6, **dadurch gekennzeichnet, dass** der Einführabschnitt (2) eine Hülle und einen Schaft aufweist, wobei die Hülle (11) distale und proximale Endabschnitte aufweist, die den Schaft des Einführabschnitts abzudecken vermögen, und die Hülle einen Anschluss (23) an dem proximalen Endabschnitt aufweist, der Luft und Wasser durch einen Innenraum der Hülle zu dem Verbindungsmechanismus (9) hin zu fördern vermag.

8. Chirurgisches Zangensystem nach einem der Ansprüche 1 oder 7, wobei der Abdeckhüllen-Hauptkörper (46) seine Form in Übereinstimmung mit den Formen des Einführabschnitts, des Behandlungsabschnitts (10) und des Verbindungsmechanismus (9) zu verändern vermag, und der Abdeckhüllen-Hauptkörper (46) an einem Außenumfang des Verbindungsmechanismus (9) anbringbar und von diesem lösbar ist.

## Revendications

1. Pince chirurgicale (1) comprenant :
une section d'insertion (2) allongée ayant des parties d'extrémité distale et proximale ;
un mécanisme de liaison (9) prévu au niveau de la partie d'extrémité distale de la section d'insertion ;
une partie de traitement (10) qui est couplée au mécanisme de liaison et mobile par l'opération du mécanisme de liaison (9), dans laquelle la partie de traitement (10) comprend une paire de mâchoires (30) ;
une section opérationnelle (3) qui est prévue au niveau de la partie d'extrémité proximale de la section d'insertion et qui est adaptée à déplacer le mécanisme de liaison par commande à distance ; et
une gaine de recouvrement (45) comprenant :
un corps principal (46) de gaine de recouvrement prévu de façon détachable de la pince (1) et adapté à recouvrir au moins le mécanisme de liaison (9) sur la pince (1),
**caractérisée en ce que** le corps principal (46) de gaine de recouvrement comprend une partie de fixation d'embout (47) à une extrémité distale de celui-ci, dans laquelle la partie de fixation d'embout (47) comprend :
une partie ramifiée (53) ramifiée en deux et adaptée à recouvrir des parties d'extrémité proximale de la paire de mâchoires (30) de la partie de traitement (10), et
une partie correspondante de mâchoire (50) s'étendant à partir de la partie ramifiée (53) et adaptée à recouvrir au moins des parties d'extrémité proximale des mâchoires (30) de la partie de traitement (10), dans laquelle la partie correspondante de mâchoire (50) comprend :
au moins deux ouvertures (52a, 52b) ayant des surfaces périphériques internes adaptées à être en contact étroit avec des surfaces périphériques extérieures des mâchoires (30), dans laquelle une longueur périphérique interne des ouvertures (52a, 52b) est formée plus courte qu'une longueur périphérique extérieure des mâchoires (30).

2. Pince chirurgicale (1) selon la revendication 1, **caractérisée en ce que** le corps principal (46) de gaine de recouvrement a une flexibilité et une élasticité.

3. Pince chirurgicale (1) selon la revendication 1 ou 2, **caractérisée en ce que** le corps principal (46) de gaine de recouvrement est formée de matériaux à base de résine.

4. Pince chirurgicale (1) selon la revendication 3, **caractérisée en ce que** les matériaux à base de résine sont un polyuréthane.

5. Pince chirurgicale (1) selon la revendication 1, dans laquelle une distance de la partie ramifiée (53) jusqu'aux ouvertures est formée plus courte que la longueur totale des mâchoires (30).

6. Pince chirurgicale (1) selon la revendication 1, **caractérisée en ce que** le corps principal (46) de gaine de recouvrement inclut un organe de préhension (48) en forme d'anneau à une extrémité proximale de celui-ci qui est adapté à être tenu par un opérateur et à engager par frottement la section d'insertion allongée.

7. Pince chirurgicale (1) selon l'une des revendications 1 à 6, **caractérisée en ce que** la section d'insertion (2) inclut une gaine et un axe, la gaine (11) ayant des parties d'extrémité distale et proximale qui sont adaptées à recouvrir l'axe de la section d'insertion, et la gaine inclut un orifice (23) au niveau de la partie d'extrémité proximale adapté à transporter de l'air et de l'eau vers le mécanisme de liaison (9) à travers un espace intérieur de la gaine.

8. Pince chirurgicale (1) selon l'une des revendications 1 à 7, dans laquelle le corps principal (46) de gaine de recouvrement est adapté à changer de forme en fonction de formes de la section d'insertion (2), de la partie de traitement (10) et du mécanisme de liaison (9), et le corps principal (46) de gaine de recouvrement peut être fixé à une, et détaché d'une, périphérie extérieure du mécanisme de liaison (9).
